# EUROPÄISCHE PATENTSCHRIFT

(11) **EP 3 956 009 B1**
(45) Veröffentlichungstag und Bekanntmachung des Hinweises auf die Patenterteilung: **04.06.2025**
(21) Anmeldenummer: 20720037.9
(22) Anmeldetag: 17.04.2020
(51) Int. Cl.: A61M 39/28, A61M 1/16, A61M 1/36, A61M 60/113, A61M 60/279, A61M 60/37, A61M 60/853

(54) **BLOCKIERVORRICHTUNG FÜR EINE SCHLAUCHKLEMME EINER BLUTBEHANDLUNGSMASCHINE**
BLOCKING DEVICE FOR A TUBE CLAMP OF A BLOOD TREATMENT DEVICE
DISPOSITIF DE BLOCAGE POUR UNE PINCE À TUYAU D'UNE MACHINE DE TRAITEMENT DU SANG

(30) Priorität: 18.04.2019 DE 102019110276
(43) Veröffentlichungstag der Anmeldung: 23.02.2022
(73) Patentinhaber: Fresenius Medical Care Deutschland GmbH, 61352 Bad Homburg (DE); Fresenius Medical Care AG, 61352 Bad Homburg (DE)
(72) Erfinder: LINDEMANN, Robert, 65193 Wiesbaden (DE); FISCHER, Gerome, 99947 Weberstedt (DE)
(74) Vertreter: Herrmann, Uwe
(86) Internationale Anmeldenummer: PCT/EP2020/060877
(87) Internationale Veröffentlichungsnummer: WO 2020/212579

(56) Entgegenhaltungen:
- DE-A1- 102006 035 404
- DE-A1- 102015 122 347
- US-A- 6 044 691
- US-A1- 2007 270 765
- US-A1- 2013 030 347
- US-A1- 2014 263 018

## Beschreibung

Die vorliegende Erfindung betrifft eine Blockiervorrichtung für eine Schlauchklemme für eine Blutbehandlungsmaschine, eine Blutbehandlungsmaschine mit einer derartigen Blockiervorrichtung sowie ein Verfahren, bei dem eine erfindungsgemäße Blockiervorrichtung zum Einsatz kommt.

Herkömmlicherweise besitzen Blutbehandlungsmaschinen als Sicherheitsmerkmal mindestens eine Schlauchklemme, insbesondere eine elektromagnetisch angetriebene Schlauchklemme, die insbesondere zum Abklemmen eines Schlauchs des extrakorporalen Blutkreislaufs dient. Die Klemme ermöglicht es, den Patienten im Verlauf der Behandlung vom extrakorporalen Blutkreislauf zu trennen, insbesondere um einen Blutverlust zu vermeiden. Aus Sicherheitsgründen ist es bekannt, die Klemme so auszulegen, dass sie im stromlosen Zustand, der beispielweise durch einen Stromausfall hervorgerufen werden kann, schließt und so den Patienten vom extrakorporalen Blutkreislauf trennt.

Im herkömmlichen Dreischichtbetrieb von Blutbehandlungszentren und beispielsweise Dialysezentren werden die Blutbehandlungsmaschinen nach der letzten Behandlung am Abend bereits für den Folgetag vorbereitet und der als extrakorporaler Blutkreislauf dienende Schlauchsatz wird an der Blutbehandlungsmaschine angeordnet und in die Schlauchklemme eingelegt.

Da sich das die Blutbehandlungsmaschine über Nacht aber regelmäßig im stromlosen Zustand befindet, klemmt die Klemme regelmäßig über einen längeren Zeitraum den Schlauch. Dies führt zu einer dauerhaften Quetschung des Schlauchs, der dadurch in dem betroffenen Bereich irreversibel verformt wird. Dies kann zu unerwünschten Turbulenzen im Betrieb führen.

Der vorliegenden Erfindung liegt die Aufgabe zugrunde, diesen Nachteil zu vermeiden.

Diese Aufgabe wird durch den Gegenstand der unabhängigen Ansprüche gelöst. Weitere vorteilhafte Weiterbildungen der Erfindung sind Gegenstand der Unteransprüche.

Eine erfindungsgemäße Blockiervorrichtung für eine Schlauchklemme einer Blutbehandlungsmaschine ist dazu ausgelegt, in der Schlauchklemme bei in der Schlauchklemme eingelegtem Schlauch derart angeordnet zu werden, dass die Schlauchklemme in einer offenen Position gehalten wird, ohne dass der in der Schlauchklemme eingelegte Schlauch durch die Schlauchklemme und / oder die Blockiervorrichtung eingeklemmt wird. Vorzugsweise ist die Klemmkraft der Schlauchklemme auf den Schlauch derart, dass der Schlauch bis zu ca. 20%, vorzugsweise nur bis zu 10% seines Außendurchmessers verformt wird. Dies ermöglicht es, dass der Schlauch fest genug gehalten wird, dass der Schlauch über Nacht nicht abrutscht, ohne dass dabei die Funktionstüchtigkeit des Schlauchs bzw. die Patientensicherheit eingeschränkt wird.

Der Kerngedanke der Erfindung besteht somit darin, dass, nachdem die Blutbehandlungsmaschine nach der letzten Behandlung am Abend bereits für den Folgetag vorbereitet und der als extrakorporaler Blutkreislauf dienende Schlauchsatz an der Blutbehandlungsmaschine angeordnet und in die Schlauchklemme eingelegt wurde, eine Blockiervorrichtung in die Schlauchklemme eingebracht wird, welche verhindert, dass die Schlauchklemme über nach den Schlauch einklemmt und somit verformt.

Bei eingesetzter Blockiervorrichtung ist die Blutbehandlungsmaschine für die nächste Blutbehandlung vorbereitet und einsatzbereit (die Schlauchleitungen für die nächste Blutbehandlung sind bereits an der Blutbehandlungsmaschine angebracht).

Vor dem eigentlichen Start der Blutbehandlung muss somit lediglich die Blockiervorrichtung aus der Schlauchklemme entfernt werden, sodass sich die Schlauchklemme wieder schließen kann. Durch die Anwesenheit der Blockiervorrichtung in der Schlauchklemme über Nacht wird ein unerwünschtes Verformen des Schlauchs verhindert und die neue Blutbehandlung kann dennoch am nächsten Morgen zügig und ohne Zeitverluste aufgrund eines Vorbereitens der Blutbehandlungsmaschine gestartet werden.

Es hat sich als vorteilhaft erwiesen, wenn die erfindungsgemäße Blockiervorrichtung einen plattenförmigen, vorzugsweise im Wesentlichen u-förmigen Grundkörper, welcher dazu ausgelegt ist, in die Schlauchklemme eingeführt zu werden, und einen vorzugsweise an einem Scheitelpunkt des im Wesentlichen u-förmigen Grundkörpers angeordneten Adapterabschnitt aufweist, mittels dessen die Blockiervorrichtung mit einer weiteren Komponente, vorzugsweise einem Dialysator, insbesondere einer Kappe eines Dialysators, verbindbar ist.

Zum Einsatz mit einer Blutbehandlungsmaschine, welche mehrere Schlauchklemmen aufweist, ist die Blockiervorrichtung vorzugsweise mittels eines Kabels, Seils oder Bands mit mindestens einer weiteren Blockiervorrichtung verbunden. Dies ermöglicht es, dass ein Benutzer mit nur einem Handgriff an das Kabel, Seil oder Band sämtliche derart miteinander verbundenen Blockiervorrichtungen beispielsweise von einer Blutbehandlungsmaschine entfernen kann. Zudem wird verhindert, dass einzelne Kleinteile verloren gehen. Die Anzahl an derart miteinander verbundenen Blockiervorrichtungen ist beliebig.

Ferner weist eine erfindungsgemäße Blockiervorrichtung mindestens eine Kodierung auf, wobei die Kodierung vorzugsweise eine Near Field Communication (NFC)-, Radio Frequency Identification (RFID)-, Quick response (QR)-, Barcode-Kodierung oder eine Kodierung in Form eines elektrischen Leiters mit einem vorbestimmten elektrischen Widerstand ist und dazu ausgelegt ist, von einer entsprechenden Detektionseinrichtung einer Blutbehandlungsmaschine erfasst oder ausgelesen zu werden. Vorzugsweise legt die Blutbehandlungsmaschine ausgelesene Informationen im Rahmen einer Blockchain-Kodierung ab.

Zudem kann die Blockiervorrichtung eindeutig einer vorbestimmten Schlauchklemme, beispielsweise der arteriellen oder venösen Schlauchklemme, und / oder einem vorbestimmten Schlauch oder Schlauchset, beispielsweise dem arteriellen oder venösen Zweig eines extrakorporalen Kreislaufs einer Blutbehandlungsmaschine, zugeordnet sein.

Weiterhin hat es sich als vorteilhaft erwiesen, wenn die Blockiervorrichtung einstückig und / oder als Wegwerf-Bauteil zur Einmalverwendung ausgebildet ist. Vorzugsweise ist die Blockiervorrichtung aus Kunststoff und / oder Metall gefertigt.

Weiterhin betrifft die vorliegende Erfindung eine Kappe für einen Dialysator, wobei die Kappe eine Aufnahme aufweist, welche mit mindestens einer erfindungsgemäßen Blockiervorrichtung vorzugsweise mittels eines Adapterabschnitts der Blockiervorrichtung verbindbar oder verbunden ist. In anderen Worten kann die Blockiervorrichtung an der Kappe angeordnet sein oder Teil der Kappe sein.

Die Blockiervorrichtung kann lösbar oder unlösbar mit der Kappe verbunden sein und kann auch einstückig mit der Kappe ausgebildet sein.

Weiterhin kann die Kappe an einem Dialysator angeordnet oder verschraubt sein. Die an der Kappe angeordnete Blockiervorrichtung kann hierbei als Griffabschnitt dienen, mittels dessen die Kappe von dem Dialysator entfernt oder abgeschraubt werden kann.

Zudem betrifft die vorliegende Erfindung eine Blutbehandlungsmaschine mit einer erfindungsgemäßen Blockiervorrichtung und / oder einer erfindungsgemäßen Kappe für einen bzw. eines Dialysators.

Weiterhin kann die Blutbehandlungsmaschine mindestens eine Aufnahme aufweisen, welche dazu ausgelegt ist, eine Blockiervorrichtung aufzunehmen. Da viele Blutbehandlungsmaschinen zwei Schlauchklemmen (arterielle und venöse Schlauchklemme) aufweisen, hat es sich als vorteilhaft erwiesen, wenn eine erfindungsgemäße Blutbehandlungsmaschine zwei entsprechende Aufnahmen aufweist.

Die Anzahl der Aufnahmen ist jedoch beliebig und kann flexibel an die Anzahl an Blockiervorrichtungen bzw. Schlauchklemmen angepasst werden.

Vorzugsweise ist eine derartige Aufnahme im Wesentlichen u-förmig ausgebildet und somit an die Form einer erfindungsgemäßen Blockiervorrichtung angepasst.

Aus Gründen der verbesserten Hygiene ist vorzugsweise an einem Scheitelpunkt der im Wesentlichen u-förmigen Aufnahme eine Drainageöffnung zum Abführen von in der Aufnahme befindlicher Flüssigkeit vorgesehen.

Ein weiterer Aspekt der Erfindung betrifft ein Verfahren zur Überprüfung der Einsatzbereitschaft einer Blutbehandlungsmaschine vor einer Blutbehandlung, mit einem Schritt der Überprüfung, ob eine erfindungsgemäße Blockiervorrichtung für eine Schlauchklemme in mindestens einer Schlauchklemme der Blutbehandlungsmaschine angeordnet ist oder nicht angeordnet ist bzw. von der Schlauchklemme entfernt wurde.

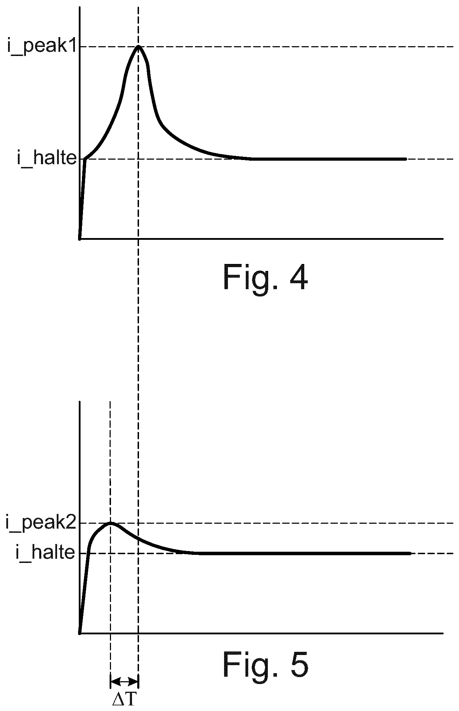

Die Überprüfung kann hierbei durch eine Messung und vorzugsweise eine darauffolgende Analyse der Stromaufnahme und / oder des Stromverlaufprofils der Schlauchklemme in Erwiderung auf eine Betätigung der Schlauchklemme erfolgen, wie dies in den Figuren Fig. 4 und 5 gezeigt ist.

Alternativ oder zusätzlich kann die Überprüfung durch eine Messung und vorzugsweise eine darauffolgende Analyse des Druckverlaufs in dem in der Schlauchklemme eingelegtem Schlauch bei aktivierter Blutpumpe der Blutbehandlungsmaschine erfolgen.

Weiterhin alternativ oder zusätzlich kann die Überprüfung durch eine Messung und vorzugsweise eine darauffolgende Analyse der Stromaufnahme und / oder des Stromverlaufprofils der Blutpumpe erfolgen

Weiterhin kann die Analyse eine Analyse des Vorzeichens einer gemessenen Druckveränderung eines Druckverlaufs umfassen, mittels welcher ermittelbar ist, ob eine im venösen Abschnitt der Blutbehandlungsmaschine angeordnete Schlauchklemme und / oder eine im arteriellen Abschnitt der Blutbehandlungsmaschine angeordnete Schlauchklemme geöffnet oder geschlossen ist. Die Blutbehandlungsmaschine weist einen extrakorporalen Kreislauf mit einem arteriellen und einem venösen Abschnitt auf. Die Blutbehandlungsmaschine weist weiterhin eine arterielle und eine venöse Schlauchklemme auf. Weiterhin kann die Blutbehandlungsmaschine einen venösen und / oder einen arteriellen Drucksensor aufweisen.

Zudem umfasst ein erfindungsgemäßes Verfahren die folgenden Schritte:
Blockieren des Starts einer Blutbehandlung, solange die Überprüfung ergibt, dass mindestens eine Blockiervorrichtung für eine Schlauchklemme in mindestens einer Schlauchklemme der Blutbehandlungsmaschine angeordnet ist, und / oder Freigeben des Starts einer Blutbehandlung, wenn die Überprüfung ergibt, dass in keiner Schlauchklemme der Blutbehandlungsmaschine eine Blockiervorrichtung angeordnet ist.

Erfindungsgemäß wird der Start einer Blutbehandlung nur dann freigegeben wenn die mindestens eine Blockiervorrichtung in einer vorbestimmten Aufnahme der Blutbehandlungseinrichtung angeordnet ist, wobei die Anwesenheit der mindestens einen Blockiervorrichtung in der Aufnahme der Blutbehandlungsmaschine mittels einer Detektionseinrichtung der Blutbehandlungsvorrichtung erfasst wird.

Weiterhin kann die Anwesenheit der mindestens einen Blockiervorrichtung in der Aufnahme der Blutbehandlungsmaschine durch das Auslesen einer Kodierung der mindestens einen Blockiervorrichtung vorzugsweise durch die Detektionseinrichtung der Blutbehandlungsvorrichtung erfasst werden, wobei die Kodierung vorzugsweise eine NFC-, RFID, QR, Barcode- Kodierung oder eine Kodierung in Form eines mit der Detektionseinrichtung der Blutbehandlungsmaschine wechselwirkenden elektrischen Leiters ist.

Weiterhin kann ein erfindungsgemäßes Verfahren den Schritt aufweisen: Abspeichern von Informationen bezüglich der mindestens einen Blockiervorrichtung unter Verwendung von Blockchain-Technologie. In anderen Worten werden Tracking-Informationen bezüglich der mindestens einen Blockiervorrichtung (oder auch mehrerer Blockiervorrichtungen) mittels Blockchain-Technologie abgelegt.

Blutbehandlungseinrichtungen werden im klinischen Alltag häufig bereits am Vorabend mit Schlauchsets oder Kassettensystemen, an welchen Schläuche angebracht sind, aufgerüstet. Die Maschine ist über Nacht stromlos, d.h. die Schlauchklemme, welche vorzugsweise elektromagnetisch betrieben wird, ist geschlossen (Normalzustand wenn stromlos) und quetscht den Schlauch über Nacht, so dass der Schlauchquerschnitt am nächsten Morgen auch bei offener Klemme verändert ist.

Die Lösung dieses Problems ist wie folgt: Eine (z.B. U-förmige) Blockiervorrichtung, vorzugsweise ausgestaltet als Klemmchip, wird in der Schlauchklemme mit dem eingelegten Schlauch eingelegt, der die Schlauchklemme in der offenen Position fixiert, ohne den Schlauch einzuklemmen.

Der Klemmchip muss vor der Behandlung entfernt werden. Die Prüfung, ob der Klemmchip entfernt wurde, kann durch Messung der Stromaufnahme bzw. des Stromverlaufsprofils bei der Betätigung der Schlauchklemme z.B. während der Durchführung eines Vorbereitungsprozesses vor der Ausführung einer Blutbehandlung erfolgen, nachdem die Blutbehandlungseinrichtung bestromt und einsatzbereit ist. Die Klemme der Schlauchklemme wird nach Inbetriebnahme der Blutbehandlungseinrichtung in die offene Position gebracht. Dabei unterscheidet sich der Stromverlauf bzw. die Stromaufnahme der elektromagnetisch betriebenen Schlauchklemme jeweils dahingehend, ob der Klemmchip sich in der Klemme befindet oder nicht, siehe Figuren 4 und 5.

Der Normalzustand der bestromten Schlauchklemme ist, dass sie offen ist. Nachdem die Blutbehandlungsmaschine bestromt und gestartet wird, wird die Schlauchklemme von der geschlossenen Position in die offene Position überführt. Dabei muss die Schlauchklemme Arbeit verrichten. Fig 4 zeigt eine hohe Amplitude (großer Peak) im Stromverlauf bei initialien Einschalten der Blutbehandlungsmaschine. Das bedeutet, dass keine Blockiervorrichtung in der Schlauchklemme steckt und die Schlauchklemme vergleichsweise hohe Arbeit verrichten muss, um die Klemme zu öffnen (Der Verfahrweg von geschlossen zu offen ist weit).

In Fig. 5 ist die Amplitude gering (kleiner Peak), da die Blockiervorrichtung in der Schlaucklemme steckt und die Schlaucklemme weniger Arbeit und geringeren Weg verrichten muss, um in die offene Position überführt zu werden, da sie ja bereits durch die Blockiervorrichtung weitestgehend offen gehalten ist. Anschließend verrichtet die Schlaucklemme Arbeit, um in der geöffneten Stellung zu verbleiben (i_halte).

Somit kann anhand des Verlaufs der Stromaufnahme der vorzugsweise elektromagnetisch betriebenen Schlauchklemme bereits bei Einschalten der Blutbehandlungsmaschine erkannt werden, ob die Blockiervorrichtung in der Schlauchklemme steckt.

Alternativ kann nach einer Inbetriebnahme die Schlauchklemme geöffnet und in die geschlossen Position überführt werden.

Auch dabei ergeben sich unterschiedliche Stromverläufe. Hierbei kann die Schlauchklemme vorteilhafterweise mehrmals öffnen und schließen, um somit ein Auswerfen einer eingelegten Blockiervorrichtung zu ermöglchen und weiters das Auswerfen anhand der Auswertung des Stromverlaufs zu erkennen

Für jeden Fall gilt, dass anhand des Stromverlaufs und/oder einer Integralrechnung auf der Grundlage der Stromaufnahmekurve und/oder eines Erkennens eines Peaks im Stromverlauf detektiert werden kann, ob eine Blockiervorrichtung / ein Klemmchip in einer Schlauchklemme angeordnet ist.

Neben der Amplitude der Peaks im Stromverlauf kann auch über die zeitliche Komponente des Stromverlaufprofils detektiert werden, ob eine Blockiervorrichtung / ein Klemmchip in einer Schlauchklemme angeordnet ist. Hierbei wird auf die Tatsache zurückgegriffen, dass der Peak im offenen und nicht blockierten Zustand zeitlich später liegt als im offenen und blockierten Zustand, da die Schlauchklemme bzw. der Hubmagnet einen längeren Weg zurücklegen muss. Somit kann auch der Zeitversatz (in Fig. 5 mit ΔT wiedergegeben) bis zum Peak ein Indiz für eine eingelegte Blockiervorrichtung / Klemmvorrichtung sein.

Eine weitere Möglichkeit zur Überprüfung der Klemmen kann über die Blutpumpe realisiert werden. Bei geschlossenen Klemmen und laufender Blutpumpe kommt es recht schnell zu einem Druckalarm (Druckanstieg oder Druckabfall), da Volumen in ein geschlossenes System hinein oder heraus gefördert wird. Der Druckalarm kann durch den venösen und / oder den arteriellen Drucksensor ausgelöst werden, welcher in der Blutbehandlungsmaschine vorgesehen ist.

Die Druckverlaufskurven bzw. der Druckverlauf in den Schläuchen bei geschlossenen und geöffneten Klemmen unterscheiden sich derart stark, dass eine Detektion zudem über die Stromaufnahme der Blutpumpe realisierbar ist.

Durch die Richtung der des Antriebsmoments der Pumpenrollen der Blutpumpe (Vorzeichen) kann detektiert werden, ob die venöse oder arterielle Klemme einzeln oder gemeinsam geöffnet sind. Die arterielle Klemme ist stromabwärts der Blutpumpe, also vor der Blutpumpe angeordnet, wobei die venöse Klemme stromaufwärts, also nach der Blutpumpe angeordnet ist.

Wenn ein Druckunterschied im Schlauch vorliegt, in anderen Worten, wenn aufgrund einer geschlossenen Klemme ein Druckunterschied an das im Schlauch befindliche Fluid angelegt, wird auf die Rollen der Blutpumpe ein Antriebsmoment ausgeübt (pro Drehung der Pumpe erfolgt mehr bzw weinger Durchsatz; bei geschlossener Klemme erfolgt weniger Durchsatz). Das Antriebsmoment ändert damit sein Vorzeichen und eine kontinuierliche Drehung ist nicht möglich. Ein konstantes Vorzeichen bedeutet bzw. bedingt also eine kontinierliche Drehung der Pumpenrollen.

In der Maschinensoftware der Blutbehandlungsmaschine ist vorzugsweise festgelegt, dass die Behandlung nicht gestartet werden kann, solange die Schlauchklemme nicht frei (von einer Blockiervorrichtung bzw. einem Klemmchip) ist.

Bei zwei oder mehr Schlauchklemmen können die Klemmchips bzw. Blockiervorrichtungen mittels eines Kabels/Seils verbunden sein, so dass sie mit einem Handgriff gehandhabt bzw. von einer Blutbehandlungsmaschine entfernt werden können.

Bei einer weiteren Variante sind die Klemmchips Teil der Abdeckkappen eines Dialysators und können gleichzeitig als "Handgriff' zum Abnehmen/Abschrauben der Kappen verwendet werden.

Bei einer Variante müssen die Klemmchips in zwei entsprechende Aufnahmen an der Blutbehandlungsmaschine eingesetzt werden, bevor die Behandlung gestartet werden kann.

Die Aufnahmen besitzen vorzugsweise eine nach unten geöffnete Öse um Flüssigkeit durch zu lassen und eine gut zu reinigende Geometrie zu bieten.

Zudem weist die Geometrie eine derartige Form auf, dass die Dialysator-Verschlussseite bzw. die dem Dialysator zugewandte Seite eines jeden Klemmchips / Blockiervorrichtung stets nach unten zeigt (in die Einführungsrichtung der Aufnahme der Blutbehandlungsmaschine) und so vor groben Schmutz und Staub weitestgehend geschützt ist.

Des Weiteren können entsprechende Kontakte in den Aufnahmen die eingesetzten Blockiervorrichtungen bzw. Klemmchips detektieren.

Erfindungsgemäß sind die Klemmchips bzw. Blockiervorrichtungen kodiert (vorzugsweise NFC, RFID, QR, Barcode, elektrischer Widerstand innerhalb eines Klemmchips oder zwischen den beiden mit einer elektrischen Leitung (mit speziellem Widerstand) verbundenen Klemmchips), vorzugsweise eindeutig einem vorbestimmten Schlauchset zugeordnet und nur einmal verwendbar ("Single Use").

Das Tracking der Blockiervorrichtungen bzw. Klemmchips und damit des Dialysators kann mittels Blockchain-Technologie erfolgen.

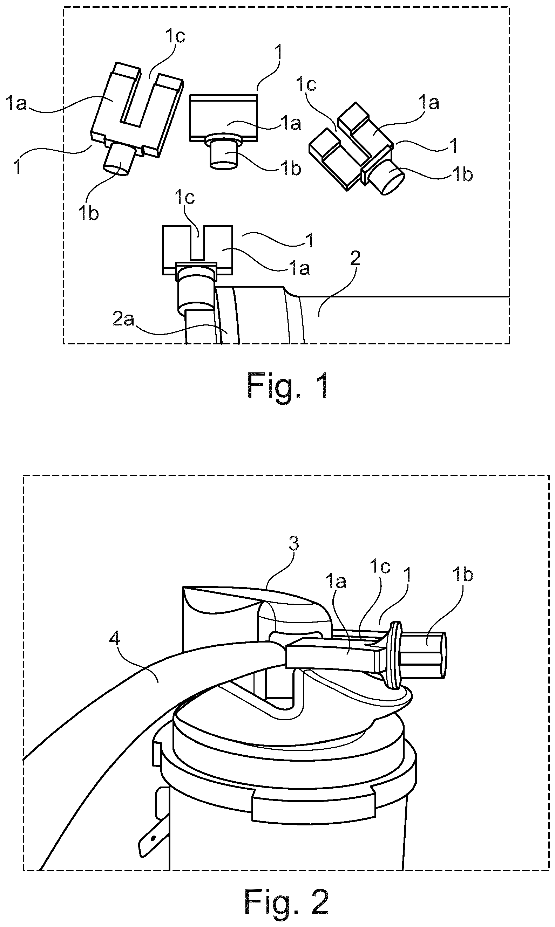

Weitere Vorteile, Merkmale und Effekte der vorliegenden Erfindungen ergeben sich aus der nachstehenden Beschreibung momentan bevorzugter Ausführungsformen unter Bezugnahme auf die Figuren 1 bis 5. Gleiche oder ähnliche Bauteile werden hierbei mit den gleichen Bezugszeichen bezeichnet.

Hierbei zeigt:
Fig. 1 mehrere verschiedene erfindungsmäße Blockiervorrichtungen;
Fig. 2 eine in einer Schlauchklemme angeordnete erfindungsmäße Blockiervorrichtung;
Fig. 3 eine an einer Kappe eines Dialysators angeordnete erfindungsmäße Blockiervorrichtung;
Fig. 4 eine Stromverlaufskurve einer Schlauchklemme ohne darin angeordnete erfindungsmäßer Blockiervorrichtung;
Fig. 5 eine Stromverlaufskurve einer Schlauchklemme mit darin angeordneter erfindungsmäßer Blockiervorrichtung .

Fig. 1 zeigt mehrere Blockiervorrichtungen 1 für eine Schlauchklemme, welche dazu ausgelegt sind, in der Schlauchklemme bei darin eingelegtem Schlauch derart angeordnet zu werden, dass die Schlauchklemme in einer offenen Position gehalten wird, ohne dass der in der Schlauchklemme eingelegte Schlauch durch die Blockiervorrichtung eingeklemmt wird.

Wie in Fig. 1 gezeigt, weist eine erfindungsgemäße Blockiervorrichtung 1 vorzugsweise einen plattenförmigen, vorzugsweise im Wesentlichen u-förmigen Grundkörper 1a, welcher dazu ausgelegt ist, in die Schlauchklemme eingeführt zu werden, und einen vorzugsweise an einem Scheitelpunkt des im Wesentlichen u-förmigen Grundkörpers 1a angeordneten Adapterabschnitt 1b auf, mittels dessen die Blockiervorrichtung 1 mit einer weiteren Komponente, vorzugsweise einem Dialysator 2, insbesondere einer Dialysatorkappe 2a, verbindbar ist.

Wie aus Fig. 1 hervorgeht, ist der plattenförmige, im Wesentlichen u-förmige Grundkörper 1a einer erfindungsgemäßen Blockiervorrichtung 1 vorzugsweise derart ausgebildet, dass in einer im Wesentlichen viereckigen Platte eine vorzugsweise schlitzförmige bzw. schartenartige Aussparung 1c ausgebildet ist.

Alternativ könnte auch ein Wesentlichen u-förmige Grundkörper 1a mit abgerundeten Ecken vorgesehen sein. Die schlitzförmige bzw. schartenartige Aussparung 1c kann durchgängig sein, sodass ein Durchgang zwischen den zwei Schenkeln des Wesentlichen u-förmige Grundkörpers 1a gebildet wird.

Alternativ dazu kann die schlitzförmige bzw. schartenartige Aussparung 1c auch lediglich die Forms eines teilweise Materialabtrags bzw. die Form einer Vertiefung aufweisen, sodass die zwei Schenkel des im Wesentlichen u-förmigen Grundkörpers 1a miteinander stofflich verbunden sind und lediglich die Dicke des plattenförmigen Grundkörpers zwischen den Schenkeln und der zwischen diesen angeordneten Aussparung 1c variiert.

Wie in Fig. 1 gezeigt, hat der Adapterabschnitt 1b eine im Wesentlichen zylindrische Form und erstreckt sich vorzugsweise rechtwinklig von einem Scheitelpunkt des Grundkörpers 1a. Wie in Fig. 1 gezeigt kann der Adapterabschnitt 1b mit einer Kappe 2a eines Dialysators 2 verbunden werden. Vorzugsweise wird der Adapterabschnitt 1b hierbei in eine entsprechende Aufnahme an der Kappe 2a eingeführt. Die Form des Adapterabschnitts 1b ist beliebig und kann an entsprechende Aufnahmen einer Kappe 2a flexibel in seiner Form angepasst werden.

Fig. 2 zeigt eine erfindungsgemäße Blockiervorrichtung 1, welche in einer Schlauchklemme 3, in welcher ein Schlauch 4 eingelegt ist, eingesetzt ist. Wie aus Fig. 2 erkennbar sind die beiden Schenkel des im Wesentlichen u-förmigen Grundkörpers 1a des Blockiervorrrichtung 1 in die Schlauchklemme 3 eingesetzt / eingeführt / eingesteckt. Die Schlauchklemme 3 wird somit in der geöffneten Position gehalten und es wird weder durch die Schlauchklemme 3 noch durch die Blockiervorrichtung 1 Druck auf den Schlauch 4 ausgeübt, welcher eine Verformung des Schlauchs 4 zur Folge hätte.

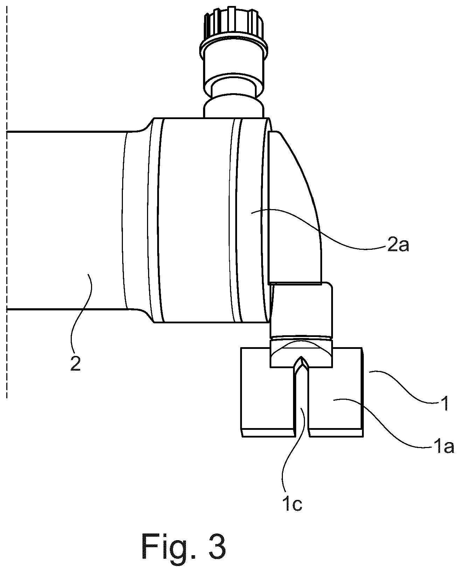

Fig. 3 zeigt eine an einer Kappe 2a eines Dialysators 2 angeordnete erfindungsmäße Blockiervorrichtung 1. Die Blockiervorrichtung ist mittels des Adapterabschnitts 1b an der Kappe 2a angebracht, indem der Adapterabschnitt 1b in eine entsprechende Aufnahme der Kappe 2a eingeführt / eingesteckt ist.

In Fig. 3 ist auch ersichtlich, inwiefern die Blockiervorrichtung 1 als Griffabschnitt dienen kann: Die Kappe 2a ist auf dem Dialysator 2 beispielsweise aufgesteckt oder aufgeschraubt und die Blockiervorrichtung 1 dient einem Benutzer, welcher die Kappe 2a von dem Dialysator 2 entfernen möchte, als Hebel und / oder als Griffstück. Die Handhabung des Dialysators wird somit durch die Blockiervorrichtung vereinfacht bzw. erleichtert.

In den Fig. 4 und 5 wird verdeutlicht, wie mittels der Erfassung und Analyse der der Stromverlaufsprofile der Schlaucklemmen überprüft werden kann, ob vor dem Start einer Blutbehandlung sämtliche etwaigen an den Schlauchklemmen der Blutbehandlungsmaschine vorhandenen Blockiervorrichtungen entfernt wurden.

Diese Überprüfung ist wichtig für die Patientensicherheit, denn nur bei entfernter Blockiervorrichtung kann eine Schlauchklemme der Blutbehandlungsmaschine im Notfall schließen, um den Patienten von dem extrakorporalen Blutkreislauf zu trennen und somit einen Blutverlust des Patienten zu verhindern.

Der Normalzustand der unbestromten Schlauchklemme ist, dass sie geschlossen ist. Der Normalzustand der bestromten Schlauchklemme ist, dass sie offen ist. Nachdem die Blutbehandlungsmaschine bestromt und gestartet wird, wird die Schlauchklemme von der geschlossenen Position in die offene Position überführt. Dabei muss die Schlauchkllemme Arbeit verrichten.

Fig. 4 zeigt eine hohe Amplitude im Stromverlauf bei initialem Einschalten der Blutbehandlungsmaschine. Das bedeutet, dass keine Blockiervorrichtung / Klemmchip steckt und die Schlauchklemme vergleichsweise hohe Arbeit verrichten muss, um die Schlauchklemme zu öffnen (Der Verfahrweg von geschlossen zu offen ist weit).

In Fig. 5 ist die Amplitude gering, da die Blockiervorrichtung / der Klemmchip in der Schlaucklemme steckt und die Schlauchklemme weniger Arbeit und geringeren Weg verrichten muss, um in die offene Position überführt zuwerden, da sie ja bereits durch die Blockiervorrichtung / den Klemmchip weitestgehend offengehalten ist.

Anschließend verrichtet die Klemme Arbeit, um in der geöffneten Stellung zu verbleiben (i_halte).

## Patentansprüche

1. Verfahren zur Überprüfung der Einsatzbereitschaft einer Blutbehandlungsmaschine vor einer Blutbehandlung, mit dem Schritt
- Überprüfung, ob eine Blockiervorrichtung für eine Schlauchklemme in mindestens einer Schlauchklemme der Blutbehandlungsmaschine angeordnet ist oder von der Schlauchklemme entfernt wurde, wobei die Blockiervorrichtung in der Schlauchklemme bei darin eingelegtem Schlauch derart angeordnet ist, dass die Schlauchklemme in einer offenen Position gehalten wird, ohne dass der in der Schlauchklemme eingelegte Schlauch durch die Schlauchklemme eingeklemmt wird;
- Blockieren des Starts einer Blutbehandlung, solange die Überprüfung ergibt, dass mindestens eine Blockiervorrichtung für eine Schlauchklemme in mindestens einer Schlauchklemme der Blutbehandlungsmaschine angeordnet ist, **dadurch gekennzeichnet, dass** der Start einer Blutbehandlung nur dann freigegeben wird, wenn die mindestens eine Blockiervorrichtung in einer vorbestimmten Aufnahme der Blutbehandlungseinrichtung angeordnet ist, wobei die Anwesenheit der mindestens einen Blockiervorrichtung in der Aufnahme der Blutbehandlungsmaschine mittels einer Detektionseinrichtung der Blutbehandlungsvorrichtung erfasst wird.

2. Verfahren nach Anspruch 1, **dadurch gekennzeichnet, dass** die Blockiervorrichtung einem plattenförmigen, vorzugsweise im Wesentlichen u-förmigen Grundkörper aufweist, welcher dazu ausgelegt ist, in die Schlauchklemme eingeführt zu werden, und dass die Blockiervorrichtung einen vorzugsweise an einem Scheitelpunkt des im Wesentlichen u-förmigen Grundkörpers angeordneten Adapterabschnitt aufweist, mittels dessen die Blockiervorrichtung mit einer weiteren Komponente, vorzugsweise einem Dialysator, insbesondere einer Kappe eines Dialysators, verbindbar ist.

3. Verfahren nach Anspruch 1 oder 2, **dadurch gekennzeichnet, dass** die Blockiervorrichtung vorzugsweise mittels eines Kabels, Seils oder Bands mit mindestens einer weiteren Blockiervorrichtung verbunden ist.

4. Verfahren nach einem der vorhergehenden Ansprüche, **dadurch gekennzeichnet, dass** die Blockiervorrichtung mindestens eine Kodierung aufweist, wobei die Kodierung vorzugsweise eine NFC-, RFID-, QR-, Barcode- Kodierung oder eine Kodierung in Form eines elektrischen Leiters mit einem vorbestimmten elektrischen Widerstand ist und vorzugsweise dazu ausgelegt ist, von einer entsprechenden Detektionseinrichtung einer Blutbehandlungsmaschine erfasst oder ausgelesen zu werden.

5. Verfahren nach einem der vorhergehenden Ansprüche, **dadurch gekennzeichnet, dass** die Blockiervorrichtung einstückig und / oder als Wegwerfbauteil ausgebildet ist.

6. Verfahren nach einem der vorhergehenden Ansprüche, **dadurch gekennzeichnet, dass** die Überprüfung durch eine Messung und vorzugsweise eine darauffolgende Analyse der Stromaufnahme und/oder des Stromprofils einer Schlauchklemme in Erwiderung auf eine Betätigung der Schlauchklemme erfolgt, und/oder die Überprüfung durch eine Messung und vorzugsweise eine darauffolgende Analyse des Druckverlaufs in dem in der Schlauchklemme eingelegten Schlauch bei aktivierter Blutpumpe erfolgt, und/oder die Überprüfung durch eine Messung und vorzugsweise eine darauffolgende Analyse der Stromaufnahme und/oder des Strromprofils der Blutpumpe erfolgt, und/oder die Überprüfung durch eine Messung und vorzugsweise eine darauffolgende Analyse des Vorzeichens eines auf mindestens eine Rolle einer Blutpumpe der Blutbehandlungsmaschine wirkenden Antriebsmoments erfolgt, mittels welcher ermittelbar ist, ob eine im venösen Kreislauf der Blutbehandlungsmaschine angeordnete Schlauchklemme und/oder eine im arteriellen Kreislauf der Blutbehandlungsmaschine angeordnete Schlauchklemme geöffnet oder geschlossen ist.

7. Verfahren nach einem der vorhergehenden Ansprüche, **dadurch gekennzeichnet, dass** die Anwesenheit der mindestens einen Blockiervorrichtung in der Aufnahme der Blutbehandlungsmaschine durch das Auslesen einer Kodierung der mindestens einen Blockiervorrichtung, wobei die Kodierung vorzugsweise eine NFC-, RFID, QR, Barcode- Kodierung oder eine Kodierung in Form eines mit der Detektionseinrichtung der Blutbehandlungsmaschine wechselwirkenden elektrischen Leiters ist, durch die Detektionseinrichtung der Blutbehandlungsvorrichtung erfolgt.

8. Blockiervorrichtung für eine Schlauchklemme, wobei die Blockiervorrichtung in der Schlauchklemme bei darin eingelegtem Schlauch derart angeordnet ist, dass die Schlauchklemme in einer offenen Position gehalten wird, ohne dass der in der Schlauchklemme eingelegte Schlauch durch die Schlauchklemme eingeklemmt wird, **dadurch gekennzeichnet, dass** die Blockiervorrichtung mindestens eine Kodierung aufweist, welche dazu ausgelegt ist, von einer entsprechenden Detektionseinrichtung einer Blutbehandlungsmaschine erfasst oder ausgelesen zu werden.

9. Blockiervorrichtung nach Anspruch 8, mit einem plattenförmigen, vorzugsweise im Wesentlichen u-förmigen Grundkörper, welcher dazu ausgelegt ist, in die Schlauchklemme eingeführt zu werden, und einem vorzugsweise an einem Scheitelpunkt des im Wesentlichen u-förmigen Grundkörpers angeordneten Adapterabschnitt, mittels dessen die Blockiervorrichtung mit einer weiteren Komponente, vorzugsweise einem Dialysator, insbesondere einer Kappe eines Dialysators, verbindbar ist.

10. Blockiervorrichtung nach Anspruch 8 oder 9, **dadurch gekennzeichnet, dass** die Blockiervorrichtung vorzugsweise mittels eines Kabels, Seils oder Bands mit mindestens einer weiteren Blockiervorrichtung verbunden ist.

11. Blockiervorrichtung nach einem der Ansprüche 8 - 10, **dadurch gekennzeichnet, dass** die Kodierung vorzugsweise eine NFC-, RFID-, QR-, Barcode- Kodierung oder eine Kodierung in Form eines elektrischen Leiters mit einem vorbestimmten elektrischen Widerstand ist und vorzugsweise dazu ausgelegt ist, von einer entsprechenden Detektionseinrichtung einer Blutbehandlungsmaschine erfasst oder ausgelesen zu werden.

12. Blockiervorrichtung nach einem der Ansprüche 8 - 11, **dadurch gekennzeichnet, dass** die Blockiervorrichtung einstückig und / oder als Wegwerfbauteil ausgebildet ist.

13. Kappe für einen Dialysator, **dadurch gekennzeichnet, dass** die Kappe eine Aufnahme aufweist, welche mit mindestens einer Blockiervorrichtung gemäß einem der Ansprüche 8 bis 12, verbunden ist.

14. Kappe für einen Dialysator nach Anspruch 13, **dadurch gekennzeichnet, dass** die Kappe an einem Dialysator angeordnet ist und die mindestens eine Blockiervorrichtung als Griffabschnitt dient, mittels dessen die Kappe von dem Dialysator entfernt oder abgeschraubt werden kann.

15. Blutbehandlungsmaschine mit einer Blockiervorrichtung gemäß einem der Ansprüche 8 bis 12 und/oder einer Kappe für einen Dialysator gemäß einem der Ansprüche 13 oder 14.

16. Blutbehandlungsmaschine nach Anspruch 15, **dadurch gekennzeichnet, dass** die Blutbehandlungsmaschine mindestens eine Aufnahme aufweist, welche dazu ausgelegt ist, eine Blockiervorrichtung aufzunehmen, und vorzugsweise im Wesentlichen u-förmig ausgebildet ist, wobei vorzugsweise an einem Scheitelpunkt der im Wesentlichen u-förmigen Aufnahme eine Drainageöffnung zum Abführen von in der Aufnahme befindlicher Flüssigkeit vorgesehen ist.

## Claims

1. A method for checking the readiness for use of a blood treatment machine prior to a blood treatment, comprising the step of
- checking whether a blocking device for a tube clamp is arranged in at least one tube clamp of the blood treatment machine or has been removed from the tube clamp, wherein the blocking device is arranged in the tube clamp, with tube placed therein, in such a way that the tube clamp is held in an open position without the tube placed in the tube clamp being clamped by the tube clamp,
- blocking the start of a blood treatment if the check reveals that at least one blocking device for a tube clamp is arranged in at least one tube clamp of the blood treatment machine, **characterised in that** the start of a blood treatment is enabled only if the at least one blocking device is arranged in a predetermined receptacle of the blood treatment device, wherein the presence of the at least one blocking device in the receptacle of the blood treatment machine is detected by means of a detection unit of the blood treatment device.

2. The method according to claim 1, **characterised in that** the blocking device has a planar, preferably substantially u-shaped main body, which is configured to be introduced into the tube clamp, and **in that** the blocking device has an adapter portion preferably arranged at an apex of the substantially u-shaped main body, by means of which adapter portion the blocking device is connectable to a further component, preferably a dialyser, in particular a cap of a dialyser.

3. The method according to claim 1 or 2, **characterised in that** the blocking device is preferably connected to at least one further blocking device by means of a cable, cord or band.

4. The method according to any one of the preceding claims, **characterised in that** the blocking device has at least one coding, wherein the coding is preferably an NFC, RFID, QR, barcode coding or a coding in the form of an electrical conductor with a predetermined electrical resistance and preferably is configured to be detected or read by a corresponding detection unit of a blood treatment machine.

5. The method according to any one of the preceding claims, **characterised in that** the blocking device is formed in one piece and/or as a throw-away component.

6. The method according to any one of the preceding claims, **characterised in that** the check is performed by a measurement and preferably a subsequent analysis of the power consumption and/or the power profile of a tube clamp in response to an actuation of the tube clamp, and/or the check is performed by a measurement and preferably a subsequent analysis of the pressure profile in the tube placed in the tube clamp when the blood pump is activated, and/or the check is performed by a measurement and preferably a subsequent analysis of the power consumption and/or the power profile of the blood pump, and/or the check is performed by a measurement and preferably a subsequent analysis of the sign of a drive moment acting on at least one roller of the blood pump of the blood treatment machine, by means of which it can be determined whether a tube clamp arranged in the venous circuit of the blood treatment machine and/or a tube clamp arranged in the arterial circuit of the blood treatment machine is open or closed.

7. The method according to any one of the preceding claims, **characterised in that** the presence of the at least one blocking device in the receptacle of the blood treatment machine is detected by the detection unit of the blood treatment device by the reading of a coding of the at least one blocking device, wherein the coding is preferably an NFC, RFID, QR, barcode coding or a coding in the form of an electrical conductor interacting with the detection unit of the blood treatment machine.

8. A blocking device for a tube clamp, wherein the blocking device is arranged in the tube clamp, with tube placed therein, in such a way that the tube clamp is held in an open position without the tube placed in the tube clamp being clamped by the tube clamp, **characterised in that** the blocking device has at least one coding which is configured to be detected or read by a corresponding detection unit of a blood treatment machine.

9. The blocking device according to claim 8, comprising a planar, preferably substantially u-shaped main body, which is configured to be introduced into the tube clamp, and an adapter portion which is preferably arranged at an apex of the substantially u-shaped main body and by means of which the blocking device is connectable to a further component, preferably a dialyser, in particular a cap of a dialyser.

10. The blocking device according to claim 8 or 9, **characterised in that** the blocking device is preferably connected to at least one further blocking device by means of a cable, cord or band.

11. The blocking device according to any one of claims 8-10, **characterised in that** the coding is preferably an NFC, RFID, QR, barcode coding or a coding in the form of an electrical conductor with a predetermined electrical resistance and preferably is configured to be detected or read by a corresponding detection unit of a blood treatment machine.

12. The blocking device according to any one of claims 8-11, **characterised in that** the blocking device is formed in one piece and/or as a throw-away component.

13. A cap for a dialyser, **characterised in that** the cap has a receptacle which is connected to at least one blocking device according to any one of claims 8 to 12.

14. The cap for a dialyser according to claim 13, **characterised in that** the cap is arranged on a dialyser and the at least one blocking device serves as a grip portion by means of which the cap can be removed or unscrewed from the dialyser.

15. A blood treatment machine with a blocking device according to any one of claims 8 to 12 and/or a cap for a dialyser according to any one of claims 13 or 14.

16. The blood treatment machine according to claim 15, **characterised in that** the blood treatment machine has at least one receptacle which is configured to receive a blocking device and is preferably substantially u-shaped, wherein a drainage opening for discharging liquid located in the receptacle is preferably provided at an apex of the substantially u-shaped receptacle.

## Revendications

1. Procédé de vérification de la disponibilité d'une machine de traitement sanguin avant un traitement sanguin, avec l'étape suivante :
- la vérification de si un dispositif de blocage pour un collier de serrage est agencé dans au moins un collier de serrage de la machine de traitement sanguin ou a été retiré du collier de serrage, le dispositif de blocage étant agencé dans le collier de serrage avec le tuyau inséré dans celui-ci de telle sorte que le collier de serrage est maintenu dans une position ouverte sans que le tuyau inséré dans le collier de serrage soit coincé par le collier de serrage ;
- le blocage du démarrage d'un traitement sanguin tant que la vérification indique qu'au moins un dispositif de blocage pour un collier de serrage est agencé dans au moins un collier de serrage de la machine de traitement sanguin, **caractérisé en ce que** le démarrage d'un traitement sanguin n'est autorisé que si l'au moins un dispositif de blocage est agencé dans un logement prédéterminé de l'appareil de traitement sanguin, la présence de l'au moins un dispositif de blocage dans le logement de la machine de traitement sanguin étant détectée au moyen d'un appareil de détection du dispositif de traitement sanguin.

2. Procédé selon la revendication 1, **caractérisé en ce que** le dispositif de blocage présente un corps de base en forme de plaque, de préférence essentiellement en forme de U, qui est conçu pour être inséré dans le collier de serrage, et **en ce que** le dispositif de blocage présente une section d'adaptateur, de préférence agencée à un sommet du corps de base essentiellement en forme de U, au moyen de laquelle le dispositif de blocage peut être relié à un autre composant, de préférence un dialyseur, notamment un capuchon d'un dialyseur.

3. Procédé selon la revendication 1 ou 2, **caractérisé en ce que** le dispositif de blocage est relié à au moins un autre dispositif de blocage, de préférence au moyen d'un câble, d'une corde ou d'une bande.

4. Procédé selon l'une quelconque des revendications précédentes, **caractérisé en ce que** le dispositif de blocage présente au moins un code, le code étant de préférence un code NFC, RFID, QR, code-barres ou un code sous forme d'un conducteur électrique ayant une résistance électrique prédéterminée et étant de préférence conçu pour être détecté ou lu par un appareil de détection correspondant d'une machine de traitement sanguin.

5. Procédé selon l'une quelconque des revendications précédentes, **caractérisé en ce que** le dispositif de blocage est réalisé d'une seule pièce et/ou sous forme de composant jetable.

6. Procédé selon l'une quelconque des revendications précédentes, **caractérisé en ce que** la vérification est effectuée par une mesure et de préférence une analyse subséquente de la consommation de courant et/ou du profil de courant d'un collier de serrage en réponse à un actionnement du collier de serrage, et/ou la vérification est effectuée par une mesure et de préférence une analyse subséquente de l'évolution de la pression dans le tuyau inséré dans le collier de serrage lorsque la pompe à sang est activée, et/ou la vérification est effectuée par une mesure et de préférence une analyse ultérieure de la consommation de courant et/ou du profil d'écoulement de la pompe à sang, et/ou la vérification est effectuée par une mesure et de préférence une analyse ultérieure du signe d'un couple d'entraînement agissant sur au moins un rouleau d'une pompe à sang de la machine de traitement sanguin, au moyen duquel il est possible de déterminer si un collier de serrage agencé dans le circuit veineux de la machine de traitement sanguin et/ou un collier de serrage agencé dans le circuit artériel de la machine de traitement sanguin est ouvert ou fermé.

7. Procédé selon l'une quelconque des revendications précédentes, **caractérisé en ce que** la présence de l'au moins un dispositif de blocage dans le logement de la machine de traitement sanguin est déterminée par la lecture, par l'appareil de détection de la machine de traitement sanguin, d'un code de l'au moins un dispositif de blocage, le code étant de préférence un code NFC, RFID, QR, code-barres ou un code sous forme d'un conducteur électrique interagissant avec l'appareil de détection de la machine de traitement sanguin.

8. Dispositif de blocage pour un collier de serrage, le dispositif de blocage étant agencé dans le collier de serrage lorsque le tuyau y est inséré, de telle sorte que le collier de serrage est maintenu dans une position ouverte sans que le tuyau inséré dans le collier de serrage soit coincé par le collier de serrage, **caractérisé en ce que** le dispositif de blocage présente au moins un code qui est conçu pour être détecté ou lu par un appareil de détection correspondant d'une machine de traitement sanguin.

9. Dispositif de blocage selon la revendication 8, avec un corps de base en forme de plaque, de préférence essentiellement en forme de U, qui est conçu pour être inséré dans le collier de serrage, et une section d'adaptateur agencée de préférence à un sommet du corps de base essentiellement en forme de U, au moyen de laquelle le dispositif de blocage peut être relié à un autre composant, de préférence un dialyseur, notamment un capuchon d'un dialyseur.

10. Dispositif de blocage selon la revendication 8 ou 9, **caractérisé en ce que** le dispositif de blocage est relié à au moins un autre dispositif de blocage, de préférence par un câble, une corde ou une bande.

11. Dispositif de blocage selon l'une quelconque des revendications 8 à 10, **caractérisé en ce que** le code est de préférence un code NFC, RFID, QR, code-barres ou un code sous forme de conducteur électrique ayant une résistance électrique prédéterminée et est de préférence conçu pour être détecté ou lu par un appareil de détection correspondant d'une machine de traitement sanguin.

12. Dispositif de blocage selon l'une quelconque des revendications 8 à 11, **caractérisé en ce que** le dispositif de blocage est réalisé d'une seule pièce et/ou sous forme de composant jetable.

13. Capuchon pour un dialyseur, **caractérisé en ce que** le capuchon présente un logement, qui est relié à au moins un dispositif de blocage selon l'une quelconque des revendications 8 à 12.

14. Capuchon pour un dialyseur selon la revendication 13, **caractérisé en ce que** le capuchon est agencé sur un dialyseur et l'au moins un dispositif de blocage sert de section de préhension au moyen de laquelle le capuchon peut être retiré ou dévissé du dialyseur.

15. Machine de traitement sanguin avec un dispositif de blocage selon l'une quelconque des revendications 8 à 12 et/ou un capuchon pour un dialyseur selon l'une quelconque des revendications 13 ou 14.

16. Machine de traitement sanguin selon la revendication 15, **caractérisée en ce que** la machine de traitement sanguin présente au moins un logement qui est conçu pour recevoir un dispositif de blocage et est réalisé de préférence essentiellement en forme de **U,** un orifice de drainage étant de préférence prévu à un sommet du logement essentiellement en forme de U pour évacuer le liquide présent dans le logement.
